# FASCICULE DE BREVET EUROPEEN

(11) **EP 2 609 852 B1**
(45) Date de publication et mention de la délivrance du brevet: **10.09.2014**
(21) Numéro de dépôt: 12358006.0
(22) Date de dépôt: 26.12.2012
(51) Int. Cl.: A61B 3/11, A61B 3/113, A61B 5/00, A61B 3/10

(54) **Procédé et dispositif de surveillance de la pupille**
Verfahren und Vorrichtung zur Beobachtung der Pupille
Method and apparatus for monitoring the pupil

(30) Priorité: 29.12.2011 FR 1104137
(43) Date de publication de la demande: 03.07.2013
(73) Titulaire: IDMED, 13382 Marseille cedex 13 (FR)
(72) Inventeur: Bernert, Frédéric, 13013 Marseille (FR); Bagnol, Thierry, 13013 Marseille (FR)
(74) Mandataire: Marchand, André

(56) Documents cités:
- WO-A1-2004/069045
- WO-A2-2006/101656
- US-A- 3 769 961
- US-A- 4 007 980
- US-A- 4 169 664
- US-A1- 2004 027 536

## Description

### DOMAINE TECHNIQUE

La présente invention est relative à un procédé et à un dispositif de surveillance de la pupille d'un sujet humain.

### ETAT DE LA TECHNIQUE

Il est connu que la pupille d'un sujet humain est un indicateur de l'état physiologique ou psychologique du sujet.

On constate en particulier que la taille de la pupille varie en fonction de nombreux paramètres de l'environnement du sujet et des stimulations extérieures, ainsi que de l'état du sujet, tels que la luminosité ambiante, la sensibilité à la douleur, l'absorption de drogues ou d'antalgiques, l'état d'éveil ou l'état émotionnel. Les réflexes de dilatation et de contraction de la pupille sont également dépendants de l'état du sujet, notamment des pathologies du sujet.

La mesure de la taille de la pupille présente ainsi un intérêt dans des domaines variés de la médecine tels que l'addictologie, l'anesthésie, l'ophtalmologie, la neurologie, la psychologie, la pharmacologie, la réanimation, ou la toxicologie.

Les mesures de la pupille peuvent être réalisées à l'aide d'un pupillomètre programmé pour mesurer les variations de taille ou de forme de la pupille, sans mettre d'objet en contact avec le globe oculaire, comme décrit par exemple dans la demande de brevet US2009/0174865.

Pour faire une telle mesure, on maintient ouvertes les paupières du sujet et on acquiert des images de l'oeil du sujet que l'on peut stimuler par des stimulations lumineuses, émotionnelles, ou douloureuses, puis on détermine la taille (surface ou diamètre par exemple) de la pupille en fonction de ces images.

Ce type d'appareil ne peut être utilisé que pendant des périodes de courte durée (quelques minutes). On ne peut en effet maintenir l'oeil ouvert pendant une plus longue période, sans risquer de dessécher l'oeil.

Les brevets US5297554 et US5903333 décrivent des appareils de mesure de la taille de la pupille qui comportent une lentille de contact oculaire solidaire d'une extension tubulaire permettant de filmer l'oeil à l'aide d'un appareil optique séparé.

Le brevet US4007980 décrit un appareil qui comporte un corps transparent adhérant à l'oeil pour suivre les mouvements de l'oeil. A ce corps sont fixées des LEDs de stimulation émettant une lumière visible, des LEDs d'éclairage de l'iris émettant une lumière invisible (infrarouge), et des photodiodes sensibles à la lumière invisible pour délivrer un signal en fonction duquel est déterminée la taille de la pupille.

L'usage de tels appareils présente également l'inconvénient que l'oeil est maintenu ouvert, ce qui va à l'encontre des pratiques médicales de surveillance de sujets inconscients. En outre, l'apport de larmes artificielles peut être rendu nécessaire, ce qui complique la mise en oeuvre de la surveillance.

### EXPOSÉ DE L'INVENTION

Un objectif de l'invention est de proposer un capteur et un dispositif incluant ce capteur, qui permette(nt) de surveiller notamment les variations - au cours du temps - de la taille de la pupille d'un sujet humain, de façon simple, confortable et sans danger pour le sujet, pendant une durée prolongée (de l'ordre d'une ou plusieurs heures au moins, par exemple de l'ordre d'un ou plusieurs jours).

Un objectif de l'invention est de proposer un capteur de surveillance de la pupille d'un sujet, un dispositif incluant ce capteur et un procédé de surveillance, qui soi(en)t amélioré(s) et/ou qui remédie(nt), en partie au moins, aux lacunes ou inconvénients des systèmes connus de surveillance de la pupille.

Selon un aspect de l'invention, il est proposé un capteur agencé pour reposer sur la cornée et/ou la sclère d'un oeil d'un sujet et sous la (ou les) paupière(s) recouvrant l'oeil, et pour délivrer, au travers de la (ou des) paupière(s), des signaux caractéristiques notamment de la taille de la pupille de l'oeil.

Selon un autre aspect de l'invention, il est proposé un dispositif de surveillance de la pupille d'un oeil d'un sujet, qui comporte un capteur adapté pour être inséré entre le globe oculaire de l'oeil et la paupière supérieure de l'oeil - ou les paupières -, une unité de traitement de signaux délivrés par le capteur qui est agencée pour déterminer notamment la taille de la pupille de l'oeil en fonction de ces signaux, et des moyens de transport de signaux au travers de la (ou des) paupière(s), qui relient le capteur à l'unité de traitement de signaux en permettant de maintenir fermées les paupières du sujet.

Selon un autre aspect de l'invention, il est proposé un procédé de surveillance de la pupille d'un sujet, dans lequel on dispose un capteur d'observation de la pupille entre la cornée et la (ou les) paupière(s) recouvrant la cornée, on alimente le capteur au travers de la (ou des) paupière(s), et on recueille les signaux d'observation de la pupille délivrés par le capteur, au travers de la (ou des) paupière(s).

A cet effet, le capteur comporte des détecteurs photoélectriques - ou photorécepteurs -, en particulier des détecteurs sensibles au rayonnement infrarouge, qui sont agencés pour « observer » la pupille, c'est-à dire pour être sensibles à la lumière réfléchie par la partie de l'iris entourant la pupille.

Les détecteurs photoélectriques - ou photorécepteurs - peuvent être des photodiodes, des phototransistors, ou des capteurs CMOS ou CCD. Ils peuvent être regroupés en grand nombre pour former un imageur matriciel, ou être des composants isolés (discrets).

Le capteur comporte un dispositif de transmission de signaux prévu pour transmettre des signaux issus des photorécepteurs, au travers de la (ou des) paupière(s), qui comporte des conducteurs électriques reliés aux photorécepteurs.

Ces conducteurs électriques peuvent être disposés dans une enveloppe étanche à l'eau ou peuvent être couverts d'un revêtement étanche à l'eau.

Les conducteurs électriques servent à transmettre l'énergie électrique nécessaire à l'alimentation des photorécepteurs ainsi que les signaux délivrés par les photorécepteurs.

Le capteur comporte un corps de forme aplatie renfermant les photorécepteurs et une partie au moins des conducteurs, dont l'épaisseur est réduite, de préférence inférieure ou égale à quatre millimètres environ, en particulier située dans une plage allant de 0,3 millimètre environ jusqu'à quatre millimètres environ.

Le corps du capteur peut présenter une forme générale de calotte sphérique comportant deux faces externes principales : une face proximale concave adaptée pour être au contact de la cornée et/ou de la sclère, et une face distale convexe adaptée pour être au contact de la paupière recouvrant le capteur et/ou la cornée.

Les parties du corps s'étendant entre les photorécepteurs et la face proximale sont généralement transparentes au rayonnement infrarouge, de sorte que lorsque l'iris est éclairé par un tel rayonnement, le rayonnement réfléchi par l'iris est transmis aux photorécepteurs par ces parties du corps, et que les signaux délivrés par les photorécepteurs en réponse à la détection du rayonnement IR réfléchi permettent de former une image de la pupille et/ou d'en mesurer le diamètre.

Le corps du capteur peut comporter deux parois superposées : une paroi distale adaptée pour supporter une portion de paupière(s), et une paroi proximale en partie au moins transparente et adaptée pour reposer sur la cornée et/ou sur la sclère.

Les photorécepteurs et conducteurs peuvent s'étendre, en partie au moins, entre ces deux parois ou dans des cavités formées dans l'une au moins de ces parois.

Chacune de ces parois peut présenter une forme générale de calotte sphérique.

La paroi proximale, qui forme la base du capteur et sert de support des photorécepteurs, peut être essentiellement constituée par une lentille de contact - ou lentille ophtalmique -, généralement non correctrice.

Cette lentille ophtalmique peut être réalisée dans un matériau perméable à l'oxygène, en particulier un matériau contenant un polymère à base de silicone, tel qu'un silicone hydrogel, pour former une lentille souple, ou bien dans un matériau plus rigide contenant par exemple du méthacrylate de méthyle, en particulier du polyméthacrylate de méthyle (PMMA).

En d'autres termes, et selon un autre aspect de l'invention, il est proposé un capteur de surveillance de l'oeil comportant une base transparente - telle qu'une lentille ophtalmique -présentant une première face concave adaptée pour reposer sur la cornée et/ou la sclère, ainsi qu'une seconde face opposée - en particulier une seconde face convexe sensiblement parallèle - à la première face concave ; le capteur comporte en outre des photorécepteurs disposés en regard de la seconde face de la base transparente, un dispositif de transmission de signaux qui est relié aux photorécepteurs, qui s'étend en regard de la base transparente et/ou le long (ou dans le prolongement) de cette base, et est agencé pour transmettre, au travers des paupières (fermées) recouvrant l'oeil, des signaux issus des photorécepteurs ; le capteur comporte également une couverture recouvrant et/ou enveloppant les photorécepteurs et une partie au moins de l'organe de transmission de signaux, la face externe (ou distale) de cette couverture étant adaptée, en particulier convexe, pour être au contact de - et/ou supporter - la face interne de la (ou des) paupière(s) fermée(s).

La couverture recouvrant et/ou enveloppant les photorécepteurs peut être transparente au rayonnement visible et/ou infrarouge ; elle peut en particulier être réalisée, comme une lentille ophtalmique, dans un matériau perméable à l'oxygène, souple ou rigide.

Alternativement, la couverture peut être opaque au rayonnement visible et/ou infrarouge.

La base transparente et/ou la couverture peu(ven)t également comporter un film de revêtement - étanche à l'eau - des photorécepteurs.

Un système optique -tel qu'une lentille - est disposé entre la face concave de la base transparente et les photorécepteurs, de façon à focaliser les photorécepteurs sensiblement dans le plan de la pupille (et de l'iris).

Le système optique peut comporter en outre un diaphragme et/ou un filtre limitant la sensibilité des photorécepteurs à des rayonnements parasites.

Ce système optique peut être solidaire des photorécepteurs, ou intégré à la base transparente.

Selon un mode de réalisation, le corps du capteur peut être essentiellement constitué d'une lentille ophtalmique et d'une couverture superposées, entre lesquelles sont insérés les photorécepteurs et une partie au moins de l'organe de transmission de signaux au travers des paupières.

Le capteur peut comporter des sources de lumière visible et/ou de rayonnement infrarouge, qui sont disposées en regard de la base transparente pour éclairer l'iris et/ou produire des stimuli visuels, de préférence à distance des photorécepteurs : les photorécepteurs sont de préférence disposés dans une partie centrale du capteur, et les sources de lumière sont disposées dans une partie périphérique du capteur.

Selon un mode de réalisation, le capteur comporte une extension prolongeant le corps du capteur et renfermant des conducteurs électriques prolongeant les conducteurs contenus dans le corps. Cette extension peut être en forme de câble en nappe et présente une épaisseur suffisamment faible pour traverser les paupières en s'étendant dans la fente séparant les paupières fermées, sensiblement sans déformer ni écarter les paupières. Cette épaisseur peut être de l'ordre de 50 microns (µm) environ à 500 µm environ.

Selon un autre mode de réalisation, l'organe de transmission de signaux du capteur comporte un module de transmission de signaux sans fil intégré dans le corps du capteur et relié aux conducteurs électriques, de façon à transmettre aux photorécepteurs des signaux d'alimentation fournis, au travers de la (des) paupière(s) recouvrant le capteur, par un appareil d'alimentation séparé du capteur, et de façon à transmettre les signaux délivrés par les photorécepteurs du capteur, à une unité de traitement de signaux séparée du capteur, au travers de la (des) paupière(s) recouvrant le capteur.

La transmission sans fil de signaux et/ou d'énergie au travers de la (des) paupière(s) peut s'effectuer notamment par ondes radio, par rayonnement infrarouge, ou par couplage inductif.

A cet effet, le capteur peut comporter une antenne incluse dans le corps du capteur et reliée aux conducteurs électriques par l'intermédiaire du module de transmission de signaux sans fil intégré au capteur.

D'autres aspects, caractéristiques, et avantages de l'invention apparaissent dans la description suivante qui se réfère aux figures annexées et illustre, sans aucun caractère limitatif, des modes préférés de réalisation de l'invention.

### BREVE DESCRIPTION DES FIGURES

La figure 1 illustre schématiquement le visage d'un sujet et un dispositif de surveillance de la pupille, dont le capteur repose sous la paupière supérieure de l'oeil du sujet.
La figure 2 illustre schématiquement, en vue en coupe transversale, un capteur de surveillance de la pupille s'étendant sous la paupière supérieure d'un oeil d'un sujet, et une partie du dispositif de surveillance incluant ce capteur.
La figure 3 illustre schématiquement, de la même façon que la figure 2, un capteur de surveillance s'étendant sous la paupière supérieure et équipé d'un organe de transmission sans fil, ainsi qu'une partie du dispositif de surveillance incluant ce capteur.
La figure 4 illustre schématiquement, en vue en coupe transversale, les principaux composants d'un dispositif de surveillance de la pupille, selon un autre mode de réalisation.
La figure 5 illustre schématiquement, en vue en coupe transversale et à échelle agrandie, les principaux composants du capteur de surveillance de l'oeil de la figure 2.
La figure 6 illustre schématiquement, en vue en plan, les principaux composants du capteur de surveillance de l'oeil de la figure 4.
La figure 7 illustre schématiquement, en vue en plan, les principaux composants d'un capteur de surveillance de la pupille, selon un autre mode de réalisation.
La figure 8 illustre schématiquement, en vue en plan, les principaux composants d'un capteur de surveillance de la pupille, selon encore un autre mode de réalisation.
La figure 8A illustre schématiquement, de la même façon que la figure 5, un capteur de surveillance de la pupille selon un autre mode de réalisation.
La figure 9 illustre schématiquement, de la même façon que les figures 7 et 8, un capteur de surveillance de la pupille selon un autre mode de réalisation.
La figure 10 illustre schématiquement, en vue en coupe transversale, un autre mode de réalisation d'un dispositif de surveillance de l'oeil comportant un capteur à transmission sans fil, avec une boucle d'induction pour la transmission d'énergie et un émetteur récepteur IR pour la transmission des signaux.
Les figures 11 à 13 illustrent une variante de réalisation dans laquelle les photorécepteurs du capteur sont disposés en ligne : la figure 11 est une vue schématique en plan ; la figure 12 est une vue schématique en coupe transversale ; et la figure 13 illustre une opération de surveillance du diamètre de la pupille à partir des données délivrées par les photorécepteurs.
Les figures 14 à 16 illustrent une variante de réalisation dans laquelle une caméra miniature et des LEDs du capteur sont disposées en ligne : la figure 14 est une vue schématique du capteur en coupe transversale ; la figure 16 est une vue schématique du capteur, en vue en plan ; et la figure 15 illustre, en vue en plan, un circuit imprimé souple commun à la caméra et aux LEDs.

### DESCRIPTION DETAILLEE DE L'INVENTION

Comme décrit plus en détail ci après, il est proposé un système de surveillance prolongée de la pupille de l'oeil qui comporte une pièce (ou base) transparente, souple ou rigide, en contact avec l'oeil, telle qu'une « lentille de contact », et un dispositif optoélectronique fixé sur cette pièce et servant à recueillir les informations optiques permettant de mesurer la pupille.

Le diamètre de la pièce transparente peut varier de 8 mm environ à 18 mm environ selon qu'elle s'appuie sur la cornée ou sur la sclère de l'oeil. Dans ce dernier cas, il peut subsister un espace entre la cornée et la pièce transparente.

Cette pièce peut intégrer la partie optique de l'imageur ou des photorécepteurs, afin d'amincir le capteur. Dans ce cas, la base transparente peut intégrer des microlentilles, des diaphragmes, et/ou des micro miroirs.

Le système comporte au moins une source de lumière visible ou dans le proche infra rouge, en particulier de longueur d'onde située dans une plage allant de 700 nanomètres (nm) environ jusqu'à 1000 nm environ, qui sert d'éclairage pour les photorécepteurs, ainsi qu'une source de stimulation lumineuse émettant une lumière blanche, rouge, verte, ou bleue. Les photorécepteurs et les sources de lumière sont orientés vers la pupille.

Le système comporte des composants électroniques servant à assurer le bon fonctionnement de l'ensemble. Ces composants peuvent comprendre des composants passifs, et éventuellement des composants actifs (circuits intégrés ou micro contrôleurs) pour la gestion de l'alimentation du capteur, pour la transmission de données et pour le traitement des signaux ou données.

Pour le confort de la paupière, les composants électriques ou électroniques sont recouverts (et protégés) par une enveloppe souple ou rigide présentant une forme ergonomique.

Le dispositif optoélectronique peut être intégré à la « lentille de contact » ou fixé de façon temporaire à cette pièce, par un adhésif faible. Dans le premier cas, le capteur est généralement à usage unique, tandis que dans le dernier cas, la lentille de contact peut être à usage unique et la partie restante du capteur peut être réutilisable.

Le capteur ainsi constitué communique avec un module extérieur, par plusieurs conducteurs, ou bien sans fil.

Dans le cas d'un capteur sans fil, l'énergie nécessaire aux composants électroniques et optoélectroniques du capteur peut être apportée par induction : un module d'alimentation placé près de la paupière émet un champ électromagnétique modulé qui est capté par une boucle d'induction intégrée au capteur, et le courant induit dans cette boucle est utilisé pour alimenter les composants du capteur.

Le capteur doit aussi échanger des données avec l'extérieur, généralement de façon bidirectionnelle.

Ces données peuvent être transmises par l'intermédiaire de la boucle d'induction ou par un canal (radio ou infrarouge) séparé.

Pour transmettre les informations par un rayonnement proche infrarouge, on utilise le fait que la paupière laisse bien passer ce rayonnement : le signal à envoyer par le capteur est délivré à une LED infrarouge intégrée au capteur et orientée vers la paupière. Un photorécepteur infrarouge placé sur un module externe proche de la paupière reçoit le signal lumineux émis par cette la LED infrarouge et ayant traversé la paupière recouvrant le capteur, et récupère alors les informations transportées par le signal.

Le fonctionnement est le même pour une communication infrarouge dans l'autre sens: une LED émettrice infrarouge est placé sur le module extérieur en direction de la paupière et un photorécepteur intégré au capteur reçois le signal lumineux ayant traversé la paupière. Les techniques de transmission peuvent être combinées en associant par exemple une transmission par induction du module extérieur vers le capteur, et une transmission par infrarouge du capteur vers le module extérieur.

Le module extérieur sert à alimenter le capteur, à piloter le capteur et à recevoir (et le cas échéant traiter) les informations issues de celui ci. Les informations recueillies sont des données d'image de la pupille ou des signaux venant des photorécepteurs placés devant la pupille et permettant d'en déterminer des caractéristiques dimensionnelles.

Des moyens de traitement de ces informations permettent de déterminer les paramètres surveillés, en particulier la taille de la pupille, et d'afficher ces paramètres via une interface utilisateur. Ces moyens de traitement peuvent être intégrés au module extérieur ou déportés dans un système séparé.

Le capteur peut être utilisé pour réaliser des mesures à diverses fréquences, par exemple à raison de plusieurs dizaines de mesures par seconde, ou à raison d'une mesure par minute. Lorsque la fréquence de mesure est faible, le capteur peut ne pas être alimenté entre deux mesures successives.

Les stimulations lumineuses du sujet peuvent être effectuées régulièrement, de façon discontinue, par exemple chaque minute, ce qui permet de tester le réflexe photo-moteur du sujet.

Dans le cas d'un capteur sans fil, le module externe de communication (émetteur/récepteur) peut être placé à proximité du capteur, par exemple sur la paupière (avec ou sans contact direct), ce qui peut permettre de ne pas intégrer de source d'éclairage et/ou de stimulation lumineuse dans le capteur sous la paupière.

Dans ce cas, par ailleurs, le module externe de communication peut former (ou être intégré à) un appareil portatif que l'on approche de la paupière du sujet pour effectuer une mesure ponctuelle.

Les composants électroniques du capteur peuvent contenir un identifiant numérique unique qui peut être transmis avec les signaux ou données de mesure afin d'identifier automatiquement le sujet de façon sûre, et d'associer les mesures effectuées au sujet concerné.

Le capteur peut être agencé pour mesurer d'autres paramètres tels que l'oxymétrie du fond de l'oeil ou la température du sujet.

On peut aussi se servir du capteur pour détecter ou mesurer un éventuel mouvement de l'oeil sous la paupière. Cela peut-être utile notamment pour prédire le réveil d'un sujet anesthésié. A cet effet, un capteur externe tel qu'une caméra peut être placé au dessus de la paupière afin de repérer la position d'une LED infrarouge solidaire du capteur placé sous la paupière. Les déplacements de cette LED infrarouge peuvent être détectés et mesurés en analysant les images délivrées par le capteur externe, ce qui informe sur les mouvements oculaires.

L'invention permet de surveiller la pupille d'un patient les yeux fermés, avec une sécurité optimale. Une fois le capteur posé sur l'oeil et la paupière fermée, aucune intervention humaine n'est nécessaire pour surveiller l'activité de l'oeil; les stimulations du sujet peuvent être des stimulations lumineuses ou douloureuses (électriques, mécaniques, ou thermiques).

Dans la description suivante, sauf indication explicite ou implicite contraire, des éléments ou organes - structurellement ou fonctionnellement - identiques ou similaires sont désignés par des repères identiques sur les différentes figures.

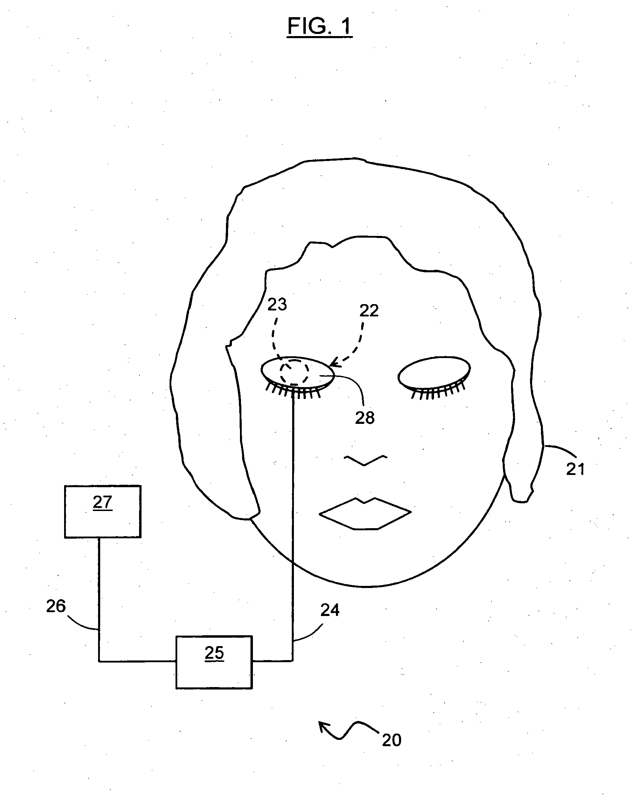

Par référence à la figure 1, un dispositif 20 est utilisé pour surveiller la dimension de la pupille de l'oeil droit 22 d'un sujet 21 inconscient dont les paupières sont fermées.

A cet effet, le dispositif 20 comporte un capteur 23 mince inséré sous la paupière 28 supérieure de l'oeil 22 et reposant sur la cornée et le cas échéant sur la sclère de l'oeil 22.

Le dispositif 20 comporte une unité 27 de traitement de signaux délivrés par le capteur 23, telle qu'un calculateur, qui est agencée -en particulier programmée - pour déterminer la taille de la pupille de l'oeil 22 en fonction de ces signaux.

Le dispositif 20 comporte en outre des moyens de transport des signaux reliant le capteur 23 à l'unité 27 de traitement de signaux et permettant de maintenir fermées les paupières du sujet.

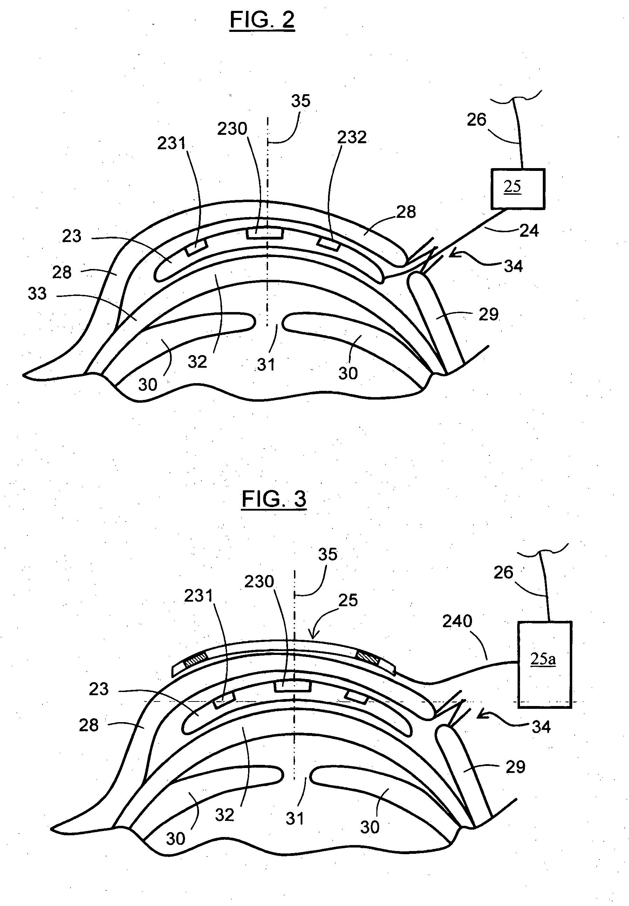

Ces moyens de transport consistent en un module d'interface 25 disposé à proximité de l'oeil 22, un câble 24 reliant le capteur 23 à l'interface 25 et s'étendant, comme illustré figure 2, dans la fente 34 séparant la paupière supérieure 28 de la paupière inférieure 29 de l'oeil 22.

Ces moyens de transport comportent en outre un câble 26 reliant l'interface 25 à l'unité de traitement 27.

Par référence aux figures 2 et 3, le capteur 23 s'étend entre la cornée 32 et la paupière supérieure 28 qui recouvre le capteur, en grande partie ou en totalité.

Selon la dimension du corps du capteur, celui-ci peut reposer sur la cornée seulement, sur la sclère 33, ou bien sur la cornée et sur la sclère.

Le corps du capteur présente une forme de calotte et une symétrie générale de révolution selon un axe 35 traversant la pupille 31 bordée par l'iris 30.

Le capteur comporte des photorécepteurs 230 disposés de façon centrale, i.e. sensiblement sur l'axe 35, des LEDs 231 de stimulation émettant dans le domaine visible - par exemple une lumière blanche -, et des LEDs 232 d'éclairage pour la mesure, qui émettent par exemple dans le proche infrarouge.

Les LEDs 231, 232 sont écartées de l'axe 35, les LEDs d'éclairage 232 pouvant être plus écartées de cet axe que les LEDs de stimulation 231.

Les récepteurs 230 sont disposés de façon à observer la zone centrale de l'oeil contenant la pupille 31, sensiblement selon l'axe 35.

Les LEDs 231 de stimulation sont disposées de façon à éclairer le fond de l'oeil, et les LEDs 232 d'éclairage sont disposées de façon à éclairer l'iris.

Dans le mode de réalisation de la figure 3 notamment, dans lequel la transmission des signaux issus des photorécepteurs du capteur 23 s'éffectue à travers la paupière 28 sans équipotentielle entre le module d'interface et le capteur, i.e. « sans fil », le module d'interface comporte un module 25 de communication avec le capteur et un module 25a de mise en forme de signaux qui est relié au module de communication par un câble 240.

Le module 25 de communication est disposé à proximité de l'oeil, en particulier sur la paupière supérieure 28 recouvrant le capteur 23.

Dans ce cas notamment, la faible distance séparant le module 25 du capteur 23 peut permettre à la fois la transmission de signaux issus des photorécepteurs du capteur, jusqu'au module 25 (donc au module 25a et à l'unité de traitement de signaux), ainsi que l'alimentation des composants du capteur par le(s) module(s) 25, 25a, au travers de la paupière 28.

Ces « communications » en champ proche entre le module 25 et le capteur 23 peuvent se faire par des ondes radio, par induction mutuelle entre des antennes (ou bobines d'antenne) respectivement intégrées au module 25 et au capteur 23, et/ou par des ondes lumineuses (infrarouge notamment) traversant la paupière 28.

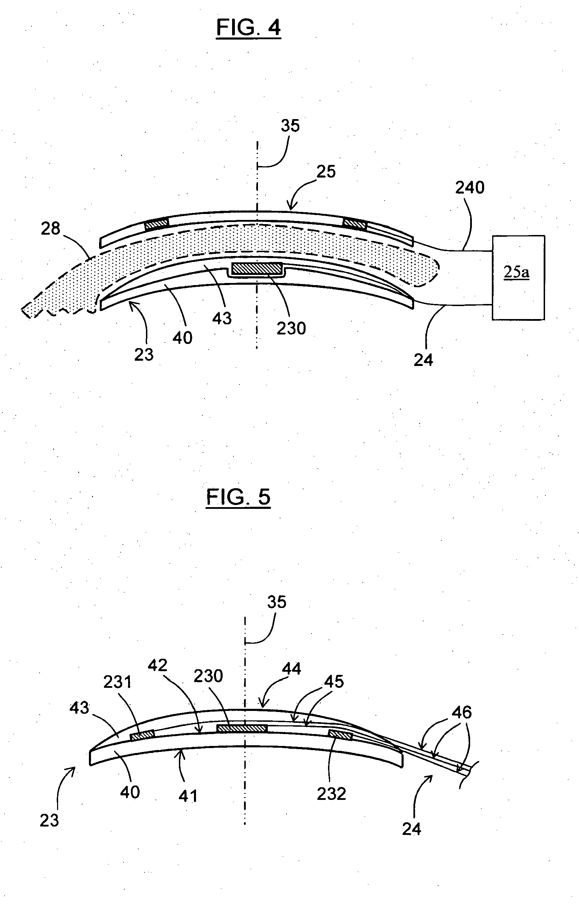

Dans le mode de réalisation illustré figure 4, le module 25a d'interface et de mise en forme de signaux est relié d'une part au capteur 23 par un câble 24 s'étendant entre les paupières, et d'autre part à un module 25 de communication reposant sur la paupière 28, à l'extérieur de celle-ci.

Dans les modes de réalisation illustrés figures 5 à 8 notamment, le capteur 23 comporte un corps 40, 43 prolongé par un câble 24 en nappe.

Le corps du capteur comporte une lentille proximale 40 - en forme de calotte - transparente au rayonnement visible et au rayonnement infra rouge, et qui s'étend entre une face proximale concave 41 et une face distale convexe 42, ces faces présentant chacune une symétrie de révolution selon l'axe 35.

Les récepteurs 230 et les LEDs 231, 232 sont disposés sur la face distale 42 de la lentille 40, et sont recouverts par une seconde lentille 43 - ou lentille distale - formant la couverture, qui peut être solidarisée à la lentille 40 par collage par exemple.

Les conducteurs 45 respectivement reliés aux LEDs et aux photorécepteurs pour leur connexion au module d'interface, sont en partie recouverts par la lentille 43.

Les parties 46 de ces conducteurs qui s'étendent à l'extérieur du corps 40, 43 du capteur, dans le prolongement du corps, sont regroupées pour former le câble 24.

Les conducteurs 46 prolongent les conducteurs 45 qui sont protégés par - ou noyés dans - la couverture 43 dont la face externe (distale) 44 est convexe.

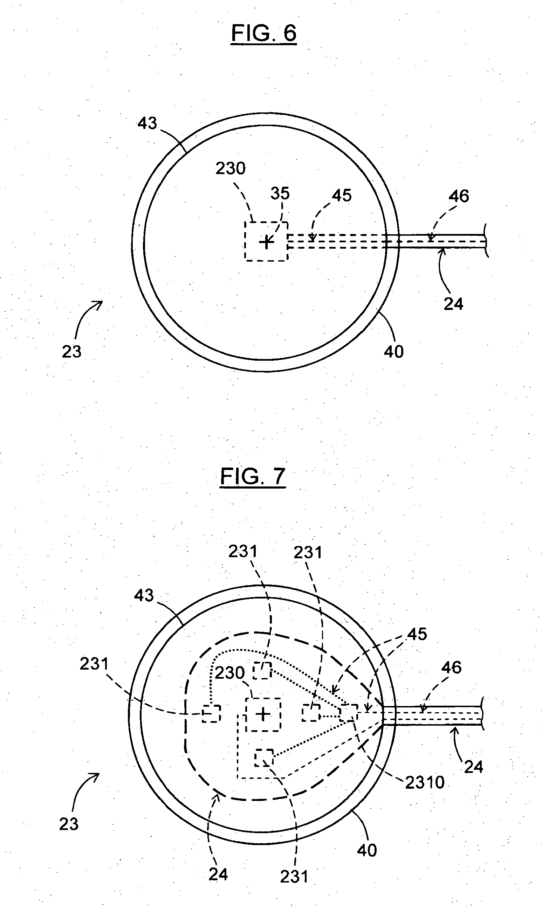

Par référence à la figure 6, le capteur 23 comporte un imageur matriciel 230 pouvant comporter plusieurs centaines de photorécepteurs - et jusqu'à un ou plusieurs million(s) de photorécepteurs (ou pixels) -, qui est centré sur l'axe 35 de symétrie du corps 40, 43 du capteur.

Dans le mode de réalisation illustré figure 7, le capteur 23 comporte un imageur matriciel 230 central et quatre LEDs 231 de stimulation qui sont alimentés par le câble 24 prolongeant le corps 40, 43, par l'intermédiaire d'un circuit 2310 de distribution relié à cet effet au module d'interface (non représenté).

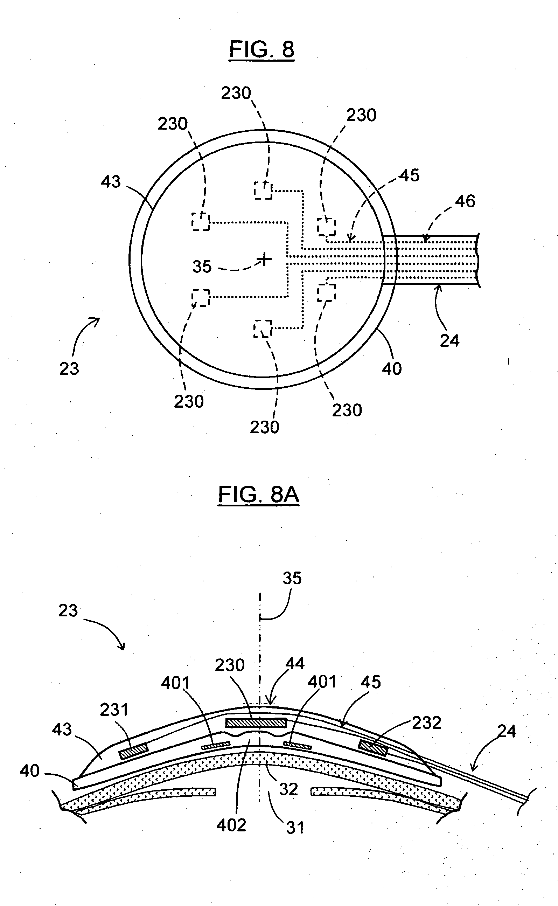

Dans le mode de réalisation illustré figure 8, le capteur 23 comporte un réseau de photorécepteurs 230 « discrets », répartis autour de l'axe 35, et à chacun desquels est associé un composant optique miniature tel qu'une lentille, de sorte que chaque photorécepteur observe une partie seulement de l'iris et de la pupille.

Par référence à la figure 8A, un diaphragme 401 et une optique 402 sont intégrés à la base 40 transparente. L'optique 402, qui est centrée sur l'axe 35 d'observation de l'imageur 230, permet de focaliser l'imageur dans le plan de la pupille 31. Le diaphragme 401, également centré sur l'axe 35, permet d'optimiser les propriétés optiques du système.

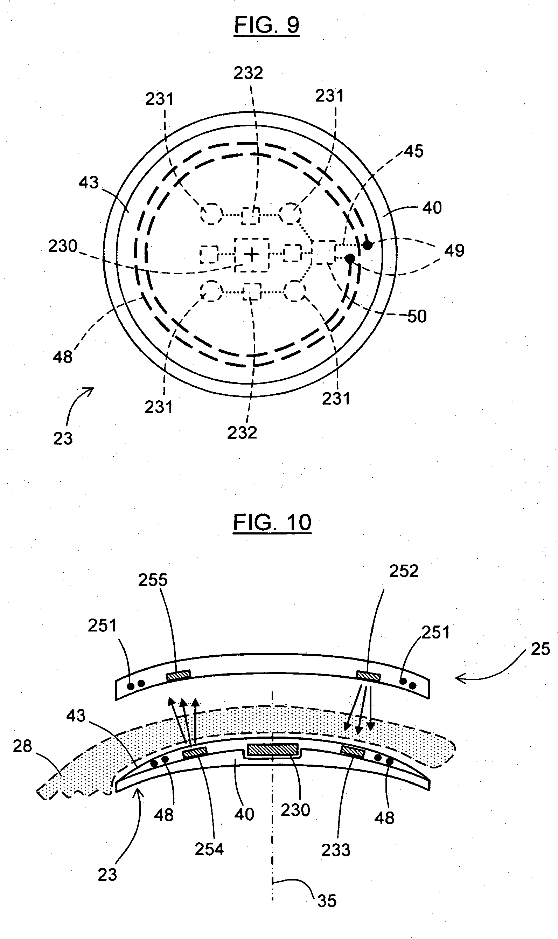

Dans le mode de réalisation illustré figure 9, le capteur 23 comporte une bobine d'antenne 48 en spirale comportant deux spires imbriquées.

Les bornes 49 de l'antenne sont reliées à un circuit intégré 50 servant à gérer la transmission de l'énergie pour l'alimentation des composants 230 à 232 du capteur, ainsi que la transmission à un module externe de communication (non représenté) des signaux ou données issus de l'imageur 230.

A cet effet, l'antenne 48, le circuit intégré 50, et les conducteurs 45 reliant le circuit 50 aux composants 230 à 232, sont intégrés au capteur et enveloppés par les parois 40, 43 formant le corps du capteur.

Par référence à la figure 10, la transmission de données et l'alimentation du capteur au travers de la paupière 28 sont réalisées par deux « canaux » différents : la transmission des données issues de l'imageur 230, jusqu'au module de communication 25 disposé au dessus de la paupière, est effectuée par des transducteurs (émetteurs et/ou récepteurs) lumineux 233, 254 intégrés au capteur 23 et protégés par la couverture 43, au travers de cette couverture et de la paupière recouvrant le capteur, tandis que l'alimentation des composants 230 à 233 est obtenue par induction, au moyen d'une bobine 48 intégrée au capteur de façon similaire à celle illustrée figure 9.

Dans ce mode de réalisation, le module 25 de communication comporte à cet effet une antenne 251 agencée pour alimenter le capteur par induction grâce à l'antenne 48, un récepteur 255 sensible au rayonnement émis par un émetteur 254 du capteur 23, et un émetteur 252 lumineux communicant avec un récepteur 233 du capteur 23.

Dans ce mode de réalisation, le dispositif de transmission de signaux comporte des émetteurs inclus dans le corps du capteur, reliés aux photorécepteurs et agencés pour échanger des signaux lumineux avec des récepteurs du module externe de communication.

La transmission de données par l'intermédiaire des transducteurs lumineux 233, 252, 254, 255 peut être obtenue par rayonnement dans une gamme de longueur d'onde pour laquelle la couverture 43 et la paupière 28 sont peu opaques, dans l'infra rouge notamment.

Dans un mode de réalisation similaire, le dispositif de transmission de signaux comporte une antenne incluse dans le corps du capteur, reliée aux photorécepteurs par des conducteurs, par l'intermédiaire d'un circuit intégré, et agencée pour échanger des signaux avec le module 25, 25a externe de communication disposé à proximité de l'oeil, sans contact (sans fil).

Dans une autre variante de réalisation dans laquelle le capteur ne comporte aucune source lumineuse, comme dans les configurations illustrées figures 6 et 8, l'alimentation des photorécepteurs et la transmission (sans fil) des signaux délivrés par les photorécepteurs peuvent s'opérer par induction ou par des émetteurs récepteurs équipant le capteur et le module 25 externe tel que celui illustré figure 10.

En outre, dans cette configuration notamment, la stimulation lumineuse de l'oeil et/ou l'éclairage de l'iris peuvent être réalisés par des sources lumineuses équipant ce module externe et produisant un flux lumineux traversant la paupière supérieure recouvrant l'oeil.

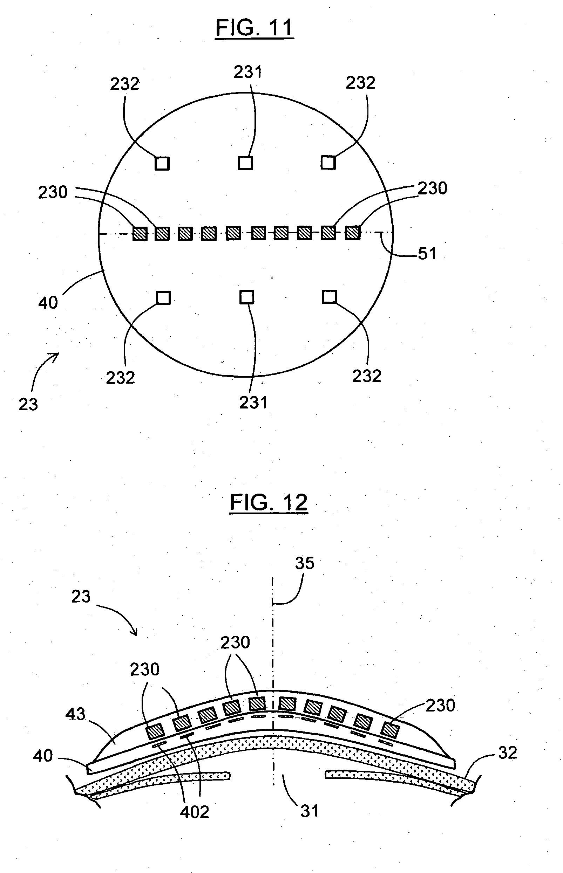

Dans la configuration illustrée figures 11 et 12, le capteur 23 comporte des photorécepteurs discrets (isolés) disposés en ligne selon le diamètre 51 d'un cercle centré sur l'axe de symétrie du corps du capteur.

Ce mode de réalisation permet de construire une « pseudo image »(image de faible résolution) à partir de l'information de luminosité délivrée par chaque photorécepteur, une micro lentille 402 étant gravée ou autrement disposée en face de chaque photodétecteur (gravée dans la lentille de contact dans l'idéal).

Ceci permet de réaliser un capteur de moindres épaisseur et consommation, et de simplifier le traitement des signaux des photorécepteurs qui peut ainsi être plus facilement réalisé par un circuit électronique intégré au capteur. Ces avantages sont notamment intéressants dans le cas d'un capteur à transmission sans fil. La précision de mesure peut cependant être moindre que celle obtenue avec un imageur matriciel.

Chaque micro lentille permet de focaliser une petite portion du plan de l'iris sur le photorécepteur associé à cette micro lentille.

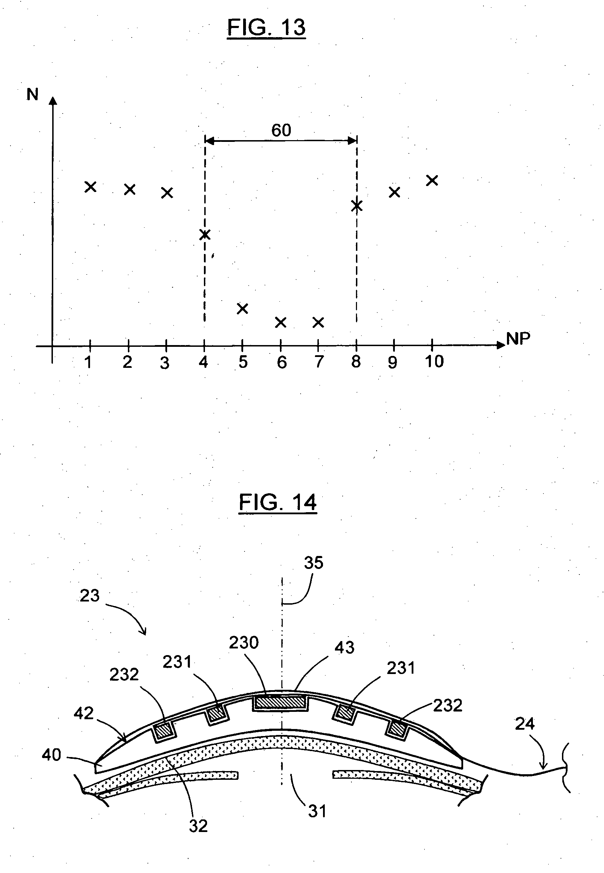

On obtient ainsi une « image » en ligne du diamètre de l'iris ce qui correspond en faite à un profil de luminosité selon un diamètre de l'iris, et qui permet d'en déduire le diamètre de la pupille, comme illustré figure 13.

Par référence à la figure 13, la position (NP) des photorécepteurs dans la « rangée » diamétrale de photorécepteurs est portée en abscisse, et le niveau (N) des signaux respectivement délivrés par les photorécepteurs est porté en ordonnée.

Les photorécepteurs placés dans les positions 1 à 4 et 8 à 10, qui observent l'iris, délivrent un signal de niveau élevé, tandis que les photorécepteurs placés dans les positions 5 à 7 qui observent la pupille délivrent un signal de plus faible niveau.

On peut ainsi estimer le diamètre 60 de la pupille à la distance séparant les photorécepteurs 4 et 8.

Dans le mode de réalisation des figures 14 à 16, un circuit imprimé souple forme un câble en nappe 24 et porte un imageur 230 et deux paires de LEDs 231, 232.

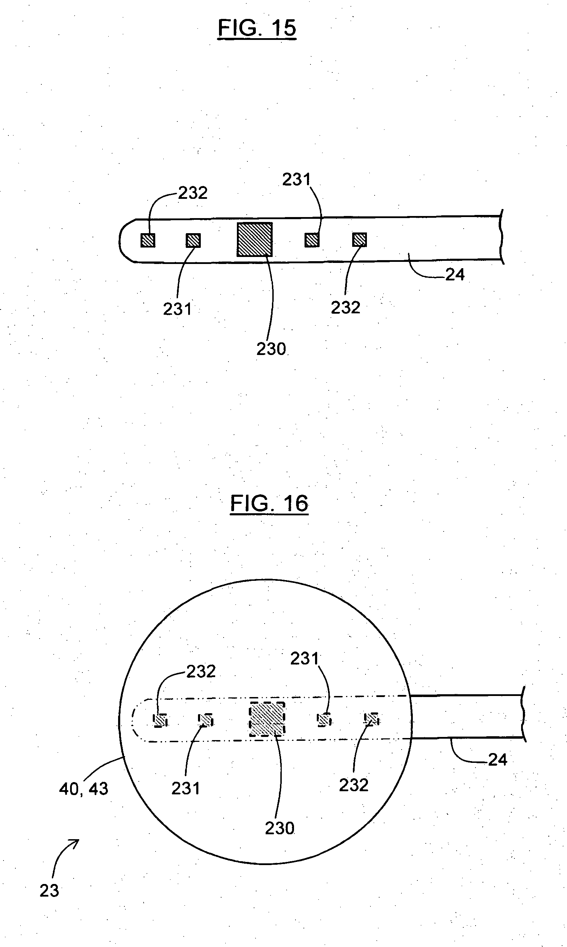

Comme illustré figure 15 et 16, ces composants sont disposés en ligne le long de l'axe longitudinal du circuit 24.

Une lentille sclérale 40 présente des cavités usinées ou moulées sur sa face distale 42, dont les dimensions et les espacements mutuels sont respectivement adaptés aux dimensions et espacements des composants 230 à 232, de sorte que ces composants peuvent être insérés dans ces cavités.

Une dépression en forme de rainure s'étend selon un diamètre de la lentille 40, sur la face distale de la lentille, et reçoit la partie du circuit imprimé souple portant les composants, la partie restante de ce circuit imprimé s'étendant (radialement) dans le prolongement de la lentille.

Un film étanche de couverture 43 recouvre la partie du circuit logée dans la dépression ainsi que les composants 230 à 232.

## Revendications

1. Capteur (23) de surveillance de la pupille (31) de l'oeil (22) qui comporte :
- une base (40) transparente présentant une première face (41) concave adaptée pour reposer sur la cornée (32) ou la sclère (33), ainsi qu'une seconde face (42) opposée à la première face;
- des photorécepteurs (230) disposés en regard de la seconde face de la base transparente, pour observer la pupille ;
- un dispositif (24, 45, 46, 48, 49, 50, 233) de transmission de signaux qui est relié aux photorécepteurs, qui s'étend en regard de la base transparente ou dans le prolongement de cette base; **caractérisé en ce que** ledit dispositif de transmission de signaux est agencé pour transmettre, au travers des paupières (28, 29) recouvrant l'oeil, des signaux issus des photorécepteurs ; et que le capteur comporte :
- une couverture (43) recouvrant les photorécepteurs et une partie au moins du dispositif de transmission de signaux, la face externe (44) de cette couverture étant adaptée pour être au contact de la (ou des) paupière(s) fermée(s).

2. Capteur selon la revendication 1 qui comporte un corps enveloppant les photorécepteurs (230) ainsi que des conducteurs (45) reliés aux photorécepteurs, le corps du capteur présentant une forme générale de calotte comportant deux faces externes principales : ladite première face (41) concave et une face distale (44) convexe adaptée pour être au contact de la (des) paupière(s) recouvrant le corps du capteur.

3. Capteur selon la revendication 1 ou 2 dans lequel la base (40) comporte une lentille ophtalmique, souple ou rigide.

4. Capteur selon l'une quelconque des revendications 1 à 3 dans lequel la base (40) et/ou la couverture (43) comporte(nt) un revêtement étanche des photorécepteurs.

5. Capteur selon l'une quelconque des revendications 1 à 4 dans lequel la couverture (43) est transparente au rayonnement visible ou infrarouge.

6. Capteur selon l'une quelconque des revendications 1 à 5 dans lequel le dispositif de transmission de signaux comporte un câble (24) en nappe prolongeant le corps du capteur et renfermant des conducteurs (46) prolongeant des conducteurs (45) contenus dans le corps, ce câble présentant une épaisseur suffisamment faible pour traverser la fente (34) formée par les paupières fermées, sensiblement sans déformer ni séparer les paupières.

7. Capteur selon l'une quelconque des revendications 1 à 6 dans lequel le dispositif de transmission de signaux comporte une antenne (48) incluse dans le corps (40, 43) du capteur et reliée aux photorécepteurs par des conducteurs (45), le cas échéant par l'intermédiaire d'un circuit intégré (50), et agencée pour échanger des signaux avec un module externe (25, 25a) de communication disposé à proximité de l'oeil.

8. Capteur selon l'une quelconque des revendications 1 à 7 dans lequel le dispositif de transmission de signaux comporte des émetteurs (233) inclus dans le corps (40, 43) du capteur, reliés aux photorécepteurs et agencés pour échanger des signaux lumineux avec des récepteurs (252) d'un module externe (25, 25a) de communication disposé à proximité de l'oeil.

9. Capteur selon l'une quelconque des revendications 1 à 8 qui comporte un système optique (401, 402) disposé entre la face concave (41) de la base transparente et les photorécepteurs (230), de façon à focaliser les photorécepteurs sensiblement dans le plan de la pupille et de l'iris, et à limiter la sensibilité des photorécepteurs à des rayonnements parasites.

10. Capteur selon l'une quelconque des revendications 1 à 9 qui comporte des sources (231, 232) de lumière visible ou de rayonnement infrarouge, qui sont disposées en regard de la base transparente pour éclairer l'iris ou produire des stimuli visuels, dans une partie périphérique du capteur, et sont couvertes par la couverture (43).

11. Capteur selon l'une quelconque des revendications 1 à 10 comportant un imageur matriciel (230) regroupant un grand nombre de photorécepteurs, et dans lequel l'épaisseur du corps (40, 43) du capteur est inférieure ou égale à quatre millimètres.

12. Dispositif (20) de surveillance de la pupille (31) d'un oeil (22) d'un sujet (21), **caractérisé en ce qu'**il comporte un capteur (23) conforme à l'une quelconque des revendications 1 à 11 et adapté pour être inséré entre l'oeil et la (ou les) paupière(s), une unité (27) de traitement de signaux délivrés par le capteur qui est agencée pour déterminer la taille de la pupille de l'oeil en fonction de ces signaux, et des moyens (24, 25, 25a, 26) de transport de signaux reliant le capteur à l'unité de traitement de signaux et permettant de maintenir fermées les paupières (28, 29) du sujet.

13. Dispositif selon la revendication 12 dans lequel les moyens de transport de signaux comportent un module externe (25) de communication sans fil disposé à proximité du capteur, en particulier sur la paupière supérieure (28) recouvrant le capteur (23), et comportant une antenne (251) et/ou des transducteurs lumineux (252, 255).

14. Procédé de surveillance de la pupille d'un sujet, dans lequel on dispose un capteur d'observation de la pupille entre la cornée et la (ou les) paupière(s) fermée(s), le capteur étant conforme à l'une quelconque des revendications 1 à 11, on alimente le capteur au travers de la (ou des) paupière(s), et on recueille les signaux d'observation de la pupille délivrés par le capteur, au travers de la (ou des) paupière(s).

## Patentansprüche

1. Sensor (23) zur Überwachung der Pupille (31) eines Auges (22), umfassend:
- einen transparente Grundplatte (40) mit einer ersten, konkaven Seite (41), dafür ausgelegt, auf der Hornhaut (32) oder der Lederhaut (33) zu ruhen, sowie einer zweiten, von der ersten Seite abgewandten Seite (42),
- Photorezeptoren (230), die der zweiten Seite der transparenten Grundplatte zugewandt angeordnet sind, zur Beobachtung der Pupille,
- eine Vorrichtung (24, 45, 46, 48, 49, 50, 233) zur Übertragung von Signalen, die mit den Photorezeptoren verbunden ist, die der transparenten Grundplatte zugewandt ist oder sich in der Verlängerung dieser Grundplatte befindet,
**dadurch gekennzeichnet, dass** die genannte Signalübertragungsvorrichtung dafür eingerichtet ist, durch die Augenlider (28, 29), die das Auge bedecken, hindurch aus den Photorezeptoren stammende Signale zu übertragen, und dass der Sensor umfasst:
- eine Abdeckung (43), die die Photorezeptoren und mindestens einen Teil der Signalübertragungsvorrichtung bedeckt, wobei die Außenseite (44) dieser Abdeckung dafür ausgelegt ist, mit dem (oder den) geschlossenen Augenlid(ern) in Berührung zu stehen.

2. Sensor nach Patentanspruch 1, der einen Körper umfasst, der die Photorezeptoren (230) umgibt, sowie Leiter (45), die mit den Photorezeptoren verbunden sind, wobei der Körper des Sensors die Gesamtform einer Kappe aufweist, die zwei Hauptaußenseiten aufweist: die genannte konkave erste Seite (41) und eine konvexe distale Seite (44), die dafür ausgelegt ist, mit dem/den Augenlid(ern) in Berührung zu stehen, das/die den Sensorkörper bedeckt/en.

3. Sensor nach Patentanspruch 1 oder 2, in dem die Grundplatte (40) eine weiche oder harte Kontaktlinse umfasst.

4. Sensor nach irgendeinem der Patentansprüche 1 bis 3, in dem die Grundplatte (40) und/oder die Abdeckung (43) eine dichte Beschichtung der Photorezeptoren umfasst (en.

5. Sensor nach irgendeinem der Patentansprüche 1 bis 4, in dem die Abdeckung (43) für Strahlung im sichtbaren oder Infrarotbereich transparent ist.

6. Sensor nach irgendeinem der Patentansprüche 1 bis 5, in dem die Signalübertragungsvorrichtung ein Flachkabel (24) umfasst, das den Sensorkörper fortsetzt und Leiter (46) enthält, die im Körper verlaufende Leiter (45) fortsetzen, wobei dieses Kabel eine ausreichend geringe Dicke aufweist, um durch den Schlitz (34) zu verlaufen, der von den geschlossenen Augenlidern gebildet wird, im Wesentlichen ohne die Lider zu verformen oder zu trennen.

7. Sensor nach irgendeinem der Patentansprüche 1 bis 6, in dem die Signalübertragungsvorrichtung eine Antenne (48) umfasst, die im Körper (40, 43) des Sensors enthalten und mit den Photorezeptoren durch Leiter (45) verbunden ist, gegebenenfalls über eine integrierte Schaltung (50), und dafür eingerichtet ist, mit einem externen Kommunikationsmodul (25, 25a), das in der Nähe des Auges angeordnet ist, Signale auszutauschen.

8. Sensor nach irgendeinem der Patentansprüche 1 bis 7, in dem die Signalübertragungsvorrichtung Sender (233) umfasst, die im Körper (40, 43) des Sensors enthalten und mit den Photorezeptoren verbunden und dafür eingerichtet sind, mit Rezeptoren (252) eines externen Kommunikationsmoduls (25, 25a), das in der Nähe des Auges angeordnet ist, Lichtsignale auszutauschen.

9. Sensor nach irgendeinem der Patentansprüche 1 bis 8, ein optisches System (401, 402) umfassend, das zwischen der konkaven Seite (41) der transparenten Grundplatte und den Photorezeptoren (230) angeordnet ist, derart dass die Photorezeptoren im Wesentlichen auf die Ebene der Pupille und der Iris fokalisiert werden und dass die Empfindlichkeit der Photorezeptoren auf Störstrahlung verringert wird.

10. Sensor nach irgendeinem der Patentansprüche 1 bis 9, der Quellen (231, 232) sichtbaren Lichts oder von Infrarotstrahlung in einem peripheren Bereich des Sensors umfasst, die der transparenten Grundplatte zugewandt angeordnet sind, um die Iris zu beleuchten oder eine visuelle Stimulation zu bewirken, und von der Abdeckung (43) bedeckt sind.

11. Sensor nach irgendeinem der Patentansprüche 1 bis 10, einen Matrix-Bilddetektor (230) enthaltend, der eine Vielzahl von Photorezeptoren umfasst und in dem die Dicke des Körpers (40, 43) des Sensors kleiner oder gleich vier Millimetern ist.

12. Vorrichtung (20) zur Überwachung der Pupille (31) des Auges (22) einer Person (21), **dadurch gekennzeichnet, dass** sie einen Sensor (23) nach irgendeinem der Patentansprüche 1 bis 11 und dafür ausgelegt, zwischen Auge und Augenlid(ern) eingesetzt zu werden, umfasst, eine Einheit (27) zur Verarbeitung von Signalen, die vom Sensor ausgegeben werden, die dafür eingerichtet ist, die Größe der Pupille des Auges aus diesen Signalen zu bestimmen, und Signaltransportmittel (24, 25, 25a, 26), die den Sensor mit der Signalverarbeitungseinheit verbinden und erlauben, die Lider (28, 29) der Person geschlossen zu halten.

13. Vorrichtung nach Patentanspruch 12, in der die Signalübertragungsmittel ein externes Funkkommunikationsmodul (25) umfassen, das in der Nähe des Sensors, insbesondere auf dem oberen Augenlid (28), das den Sensor (23) bedeckt, angeordnet ist und eine Antenne (251) und/oder Lichtwandler (252, 255) aufweist.

14. Verfahren zur Überwachung der Pupille einer Person, in dem ein Pupillenbeobachtungssensor zwischen der Hornhaut und dem (oder den) geschlossenen Augenlid(ern) angeordnet wird, wobei der Sensor irgendeinem der Patentansprüche 1 bis 11 entspricht, der Sensor durch das (oder die) Lid(er) hindurch versorgt wird und die vom Sensor ausgegebenen Pupillenbeobachtungssignale durch das (oder die) Lid(er) hindurch aufgefangen werden.

## Claims

1. A sensor (23) for monitoring the pupil (31) of an eye (22), comprising:
· a transparent base (40) presenting a concave first face (41) suitable for resting on the cornea (32) or the sclera (33), and a second face (42) opposite from the first face;
· light receivers (230) arranged facing the second face of the transparent base in order to observe the pupil;
· a signal transmission device (24, 45, 46, 48, 49, 50, 233) that is connected to the light receivers, that extends facing the transparent base or that extends said base,
**characterized in that** said signal transmission device is arranged to take signals from the light receivers and transmit them through one or both of the eyelids (28, 29) covering the eye, and **in that** the sensor comprises
· a covering (43) covering the light receivers and at least a portion of the signal transmission device, the outer face (44) of the covering being adapted to be in contact with the closed eyelid(s).

2. A sensor according to claim 1 which comprises a body covering the light receivers (230) and the conductors (45) connected to the light receivers, the body of the sensor being generally in the form of a spherical cap having two main outer faces: said concave first face (41) and a convex distal face (44) adapted to be in contact with the eyelid(s) covering the body of the sensor.

3. A sensor according to claim 1 or claim 2, wherein the base (40) comprises a soft or hard ophthalmic contact lens.

4. A sensor according to any one of claims 1 to 3, wherein the base (40) and/or the covering (43) comprise(s) a leaktight coating of the light receivers.

5. A sensor according to any one of claims 1 to 4, wherein the covering (43) is transparent to visible or infrared radiation.

6. A sensor according to any one of claims 1 to 5, wherein the signal transmission device comprises a ribbon cable (24) extending the body of the sensor and containing conductors (46) extending conductors (45) contained in the body, the cable presenting thickness that is small enough to pass through the gap (34) between the closed eyelids, substantially without deforming or separating the eyelids.

7. A sensor according to any one of claims 1 to 6, wherein the signal transmission device comprises an antenna (48) included in the body (40, 43) of the sensor and connected to the light receivers by conductors (45), where appropriate via an integrated circuit (50), and arranged to exchange signals with an external communications module (25, 25a) placed in the proximity of the eye.

8. A sensor according to any one of claims 1 to 7, wherein the signal transmission device comprises emitters (233) included in the body (40, 43) of the sensor, which emitters are connected to the light receivers and are arranged to exchange light signals with receivers (252) of an external communications module (25, 25a) arranged in the proximity of the eye.

9. A sensor according to any one of claims 1 to 8, including an optical system (401, 402) arranged between the concave face (41) of the transparent base and the light receivers (230) so as to focus the light receivers substantially on the plane of the pupil and the iris, and so as to limit the sensitivity of the light receivers to interfering radiation.

10. A sensor according to any one of claims 1 to 9, including sources (231, 232) of visible light or of infrared radiation that are arranged facing the transparent base in order to illuminate the iris or to produce visible stimuli, the sources being arranged in a peripheral portion of the sensor and being covered by the covering (43).

11. A sensor according to any one of claims 1 to 10, including a matrix imager (230) having a large number of light receivers, and wherein the thickness of the body (40, 43) of the sensor is less than or equal to four millimeters.

12. A device (20) for monitoring the pupil (31) of an eye (22) of a subject (21), the device being **characterized in that** it includes a sensor (23) according to any one of claims 1 to 11 which is adapted to be inserted between the eye and the eyelid(s), a processor unit (27) for processing signals delivered by the sensor and arranged to determine the size of the pupil of the eye as a function of these signals, and signal transport means (24, 25, 25a, 26) connecting the sensor to the signal processor unit and allowing the subject's eyelids (28, 29) to be kept closed.

13. A device according to claim 12, wherein the signal transport means comprise a wireless external communications module (25) arranged in the proximity of the sensor, in particular on the upper eyelid (28) covering the sensor (23), and including an antenna (251) and/or light transducers (252, 255).

14. A method of monitoring the pupil of a subject, wherein a sensor for observing the pupil is arranged between the cornea and the closed eyelid(s), the sensor being in accordance with any one of claims 1 to 11, the sensor is powered through the eyelid(s), and the pupil-observation signals delivered by the sensor are collected through the eyelid(s).
